# EUROPEAN PATENT APPLICATION

(11) **EP 4 796 543 A1**
(43) Date of publication of application: **26.08.2026**
(21) Application number: 25736595.7
(22) Date of filing: 06.03.2025
(51) Int. Cl.: C07D 471/04

(54) **METHOD FOR SYNTHESIZING ARYL-SUBSTITUTED CHIRAL TETRAHYDROPYRAN RING BY MEANS OF DE-SYMMETRIZATION**

(30) Priority: 08.11.2024 CN 202411589551
(71) Applicant: Allsino Pharmaceutical Co., Ltd., Hangzhou, Zhejiang 311606 (CN)
(72) Inventor: The designation of the inventor has not yet been filed
(74) Representative: Metida
(86) International application number: PCT/CN2025/081152
(87) International publication number: WO 2026/097750

(57) **Abstract**

A method for synthesizing an aryl-substituted chiral tetrahydropyran ring via desymmetrization is provided. A cyclic anhydride of formula I or a symmetric dicarboxylate of formula II undergoes catalytic alcoholysis to obtain an intermediate of formula III. The intermediate reacts with a methyl metal reagent to obtain a lactone of formula IV, which is then reduced to an ether of formula V. A compound of formula V-A undergoes a copper-catalyzed reaction to obtain a corresponding arylhydrazine of formula VI, which then undergoes indole cyclization with a pyruvic acid derivative under acid catalysis to obtain a target product. A compound of formula V-B undergoes functional group transformation to obtain an intermediate of formula VIII, which then reacts with an acrylic acid derivative to generate a target compound. According to the present invention, use of transition metal catalysts and SFC resolution reported in previous methods are avoided.

## Description

### FIELD OF TECHNOLOGY

The present invention belongs to the technical field of organic chemistry and medicinal chemistry, and relates to a method for synthesizing a chiral tetrahydropyran ring, and in particular to a method for synthesizing an aryl-substituted chiral tetrahydropyran ring via desymmetrization.

### BACKGROUND

Application background of aryl-substituted chiral tetrahydropyran rings: this structure and its derivatives, such as lactones and hemiacetals, can be further derivatized to obtain more complex molecules, among which structures with high chiral purity can serve as key synthons in the synthesis of pharmaceutical molecules. For example, Orforglipron and ECC5004, as small-molecule GLP-1 receptor agonists, exert effective antidiabetic effects by enhancing glucose-dependent insulin secretion and improving energy balance. They also exhibit pharmacokinetic properties favorable for oral administration, offering significant advantages and demonstrating substantial potential in the pharmaceutical market.

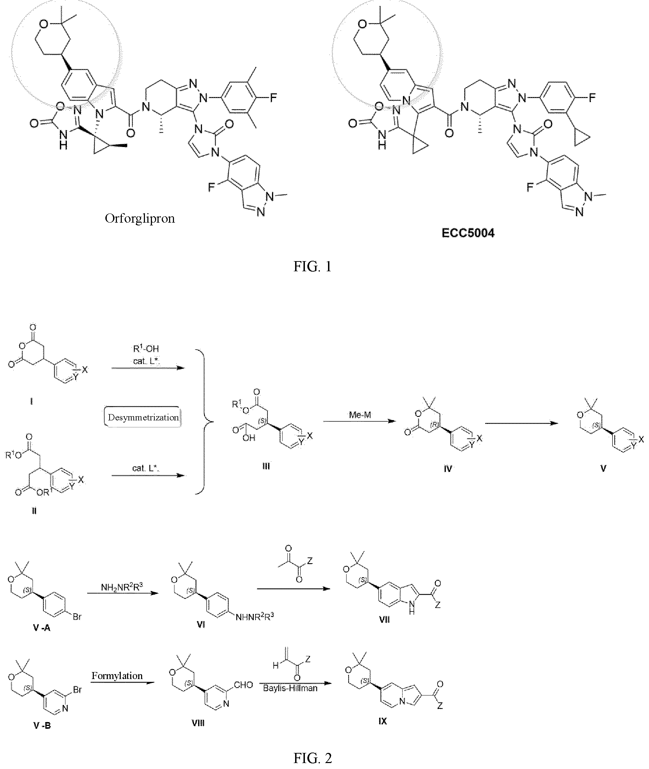

As the core module of Orforglipron and ECC5004 (see FIG. 1), and also one of the synthetic challenges, the synthesis improvement of aryl-substituted chiral tetrahydropyran ring fragments in their structures, namely (S)-5-(2,2-dimethyltetrahydro-2H-pyran-4-yl)-1H-indole-2-carboxylic acid and (S)-7-(2,2-dimethyltetrahydro-2H-pyran-4-yl)-indazine-2-carboxylic acid and derivatives thereof, is particularly important.

Currently, the construction of chiral centers in aryl-substituted chiral tetrahydropyran ring fragments mainly relies on transition metal palladium-catalyzed formation of carbon-carbon bonds between aryl groups and tetrahydropyran rings, followed by chromatographic separation or transition metal-catalyzed asymmetric hydrogenation to afford enantiomers with high chiral purity. Some methods also employ enzyme catalysis or transition metal catalysis to obtain precursor structures of aryl-substituted chiral tetrahydropyran rings from specific substrates.

At present, there are few disclosed patents on synthetic methods for aryl-substituted chiral tetrahydropyran ring fragments, with only a few original research patents involved. For example, in Patent WO2018056453A1, a substrate bromoindole undergoes a palladium-catalyzed Negishi reaction with an in-situ prepared organozinc reagent of a tetrahydropyran structure to form a carbon-carbon bond, yielding the racemate of the target structure, which is then resolved by supercritical fluid chromatography (SFC) to obtain the target structure with the desired configuration.

As another example, in patents such as WO/2022/017338 and US11584751B1, a substrate bromoindole undergoes a Pd-catalyzed Suzuki reaction with a boronic ester of a dihydropyran ring, followed by reduction of a double bond in the dihydropyran ring using Pd/C to yield the racemate of the target structure, which is finally subjected to chiral resolution via SFC to obtain the target structure with the desired stereoconfiguration. The fragment in the ECC5004 molecule is also synthesized based on this method.

In a recently published patent (CN117777111A), a raw material bromoindole undergoes a palladium-catalyzed Heck coupling with an α,β-unsaturated lactone to form a carbon-carbon bond. A key chiral center is then established through ruthenium-catalyzed asymmetric hydrogenation, followed by two additional steps to build the target molecular structure.

However, in existing methods, the use of transition metals not only increases synthesis costs but also poses the problem of metal residues in subsequent pharmaceutical synthesis. Additionally, the equipment required for SFC is relatively expensive, making it unsuitable for industrial production. Moreover, the undesired configuration is discarded, significantly increasing the synthesis cost of the target structure.

### SUMMARY

To address the aforementioned problems, the present invention provides a method for synthesizing an aryl-substituted chiral tetrahydropyran ring via desymmetrization. The present invention includes synthesizing a cyclic anhydride of formula I or a symmetric dicarboxylate of formula II with the commonly used method in the literature, which is then subjected to alcoholysis catalyzed by an ethylenediamine-based organic small molecule to obtain an intermediate of formula III with a target stereoconfiguration and a high enantiomeric excess (ee) value (or the symmetric dicarboxylate of formula II undergoes monohydrolysis under catalysis of an organic small molecule or an enzyme to obtain an intermediate of formula III with a target stereoconfiguration and a high ee value). The intermediate reacts with a methyl metal reagent to obtain a dimethylated lactone of formula IV, which is reduced to obtain an ether of formula V.

To achieve the above objective, the present invention adopts the following technical solutions:

The present invention provides a method for synthesizing an aryl-substituted chiral tetrahydropyran ring via desymmetrization, including the following steps:
a) alcoholyzing a compound of formula I through organic small-molecule catalysis or enzyme catalysis to obtain a compound of formula III with high chiral purity;
b) reacting the compound of formula III with a methyl metal reagent to obtain a compound of formula IV;
c) reducing a lactone in the compound of formula IV to obtain a compound of formula V;
d) subjecting a compound of formula V-A to a copper-catalyzed hydrazination reaction to obtain a compound of formula VI;
e) subjecting the compound of formula VI to acid-catalyzed Fisher cyclization with a pyruvic acid derivative to obtain a compound of formula VII;
or step d) being replaced with step f):
f) allowing a compound of formula V-B to be treated with a metal reagent and react with N,N-dimethylformamide to obtain a compound of formula VIII; and
g) subjecting the compound of formula VIII to cyclization with an acrylic acid derivative to obtain a compound of formula IX;
where the compound of formula I is: 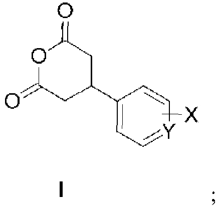
the compound of formula III is: 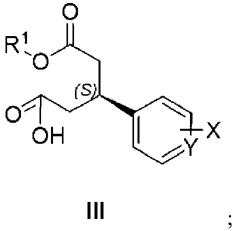
the compound of formula IV is: 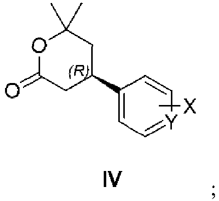
the compound of formula V is: 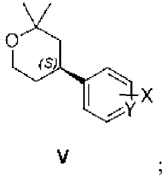
the compound of formula V-A is: 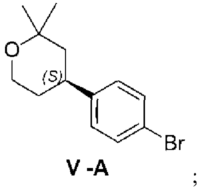
the compound of formula VI is: 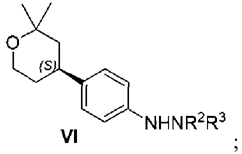
the compound of formula VII is: 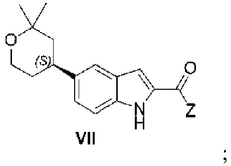
the compound of formula V-B is: 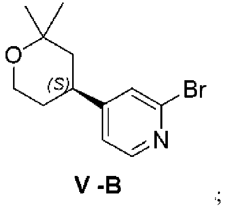
the compound of formula VIII is: 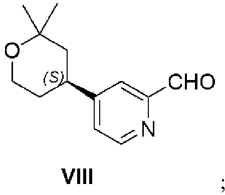 and
the compound of formula IX is: 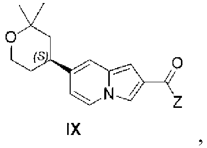
where in the formulas, X is halogen (Cl, Br, I), -OTf, -OMs, or -OTs; Y is C or N; R¹ is C1-C6 alkyl or benzyl; R² is H, Me, Et, iPr, or Bn; R³ is H or Boc; Z is -OR⁴; R⁴ is C1-C6 alkyl or -NR⁵R⁶; R⁵ is C1-C3 alkyl; and R⁶ is phenyl or substituted phenyl.

As a preferred implementation of the present invention, the step a) is replaced with step a1): monohydrolyzing a compound of formula II through organic small-molecule catalysis or enzyme catalysis to obtain the compound of formula III with high chiral purity,
the compound of formula II is: 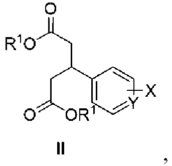
where in the formula, X is halogen (Cl, Br, I), -OTf, -OMs, or -OTs; Y is C or N; and R¹ is C1-C6 alkyl or benzyl.

As a preferred implementation of the present invention, in the step a) or the step a1), a substrate for the alcoholysis reaction includes one of methanol, ethanol, benzyl alcohol, or other alkyl alcohols, and a quantity of the substrate as used in the alcoholysis reaction is 1.0-10.0 equivalents.

As a preferred implementation of the present invention, in the step b), the methyl metal reagent includes one of methyllithium, methylmagnesium bromide, methylmagnesium chloride, or methylmagnesium iodide; and a quantity of the methyl metal reagent as used is 3.0-5.0 equivalents.

As a preferred implementation of the present invention, in the step c), a reducing agent used for reducing the lactone includes DIBAL-H and Et₃SiH.

As a preferred implementation of the present invention, in the step d), a copper catalyst includes CuI or CuBr, and a quantity of the copper catalyst as used is 0.01-1.0 equivalents.

As a preferred implementation of the present invention, in the step d), a ligand used in the reaction includes N-(2,6-dimethylphenyl)-6-hydroxypyridinecarboxamide or N1,N2-bis(2,5-dimethyl-1H-pyrrol-1-yl)oxalamide; and a substrate for the hydrazination reaction includes hydrazine hydrate, 1-tert-butoxycarbonyl-1-methylhydrazine, 1-tert-butoxycarbonyl-1-ethylhydrazine, or 1-tert-butoxycarbonyl-1-benzylhydrazine.

As a preferred implementation of the present invention, in the step e), an acid used for the acid catalysis includes HCl, PPA, TsOH, or TfOH; and the pyruvic acid derivative includes ethyl pyruvate, methyl pyruvate, or N-methyl-2-oxo-N-phenylpropanamide.

As a preferred implementation of the present invention, in the step f), the metal reagent includes n-butyllithium, sec-butyllithium, isopropylmagnesium chloride, or isopropylmagnesium bromide.

As a preferred implementation of the present invention, in the step g), the acrylic acid derivative includes ethyl acrylate, methyl acrylate, benzyl acrylate, or N-methyl-N-phenylacrylamide.

In the present invention, a cyclic anhydride of formula I or a symmetric dicarboxylate of formula II synthesized with the commonly used method in the literature is subjected to alcoholysis catalyzed by an ethylenediamine-based organic small molecule to obtain an intermediate of formula III with a target stereoconfiguration and a high enantiomeric excess (ee) value (or the symmetric dicarboxylate undergoes monohydrolysis under catalysis of an organic small molecule or an enzyme to obtain an intermediate of formula III with a target stereoconfiguration and a high ee value). The intermediate reacts with a methyl metal reagent to obtain a dimethylated lactone of formula IV, which is reduced to obtain an ether of formula V.

Further, a compound of formula V-A undergoes copper catalysis to obtain a corresponding arylhydrazine of formula VI, which finally undergoes Fisher indole cyclization with a pyruvic acid derivative under acid catalysis to obtain a target product of formula VII.

Alternatively, a compound of formula V-B undergoes functional group transformation to form an aldehyde of formula VIII, which then undergoes a Balis-Hillman reaction with an acrylic acid derivative, followed by cyclization to generate a compound of formula IX.

Compared with the prior art, the present invention has the following beneficial effects:
1) The starting materials of the present invention have simple structures and are commercially available in bulk. In the present invention, an inexpensive and readily available chiral ethylenediamine-based organic small molecule or a commercially available enzyme is used for catalysis to construct a chiral center at the 4-position of the tetrahydropyran ring via a desymmetrization method.
2) In the present invention, the desymmetrization of the substrate I or II is achieved through catalysis by the chiral ethylenediamine-based organic small molecule or the commercially available enzyme (including, but not limited to, these methods) to obtain the required key chiral center and synthesize the intermediate of formula III. This step is the key to realizing the present process route.
3) According to the present invention, use of transition metal catalysts and SFC resolution reported in previous methods are avoided, reducing reaction costs and facilitating scale-up production, which represents an economical, environmentally friendly technical route with simple post-treatment and easy for scale-up production.

### BRIEF DESCRIPTION OF THE DRAWINGS

FIG. 1 is a schematic diagram of Orforglipron and ECC5004.
FIG. 2 is a synthetic route diagram of the present invention.
FIG. 3 is a nuclear magnetic resonance spectrum of a compound of VII.
FIG. 4 is a chiral chromatogram of a racemate of a compound of VII.
FIG. 5 is a chiral chromatogram of a single chiral target of a compound of VII.

### DESCRIPTION OF THE EMBODIMENTS

The technical solutions in the examples of the present invention will be clearly and completely described below in conjunction with the examples of the present invention. Obviously, the described examples are only a part of the examples of the present invention, rather than all the examples. Based on the examples in the present invention, all other examples obtained by those of ordinary skill in the art without creative efforts shall fall within the scope of protection of the present invention.

In the present invention, the raw materials, reagents or equipment used can all be commercially available, as shown in Table 1.

**Table 1. Raw materials**

| Serial number | Material name | CAS.NO | Batch number | Manufacturer |
|---|---|---|---|---|
| 1 | Ethyl acetoacetate | 141-97-9 | P2099679 | Titan |
| 2 | p-Bromobenzaldehyde | 1122-91-4 | P1948667 | Titan |
| 3 | Acetyl chloride | 75-36-5 | P2791990 | Titan |
| 4 | Methylmagnesium chloride | 676-58-4 | 230706 | Shangyu Hualun |
| 5 | Boron trifluoride diethyl etherate | 109-63-7 | P1775474 | Titan |
| 6 | Triethylsilane | 617-86-7 | P2517733 | Titan |
| 7 | O-(7-azabenzotriazol-1-yl)-N,N,N',N'-tetramethyluronium hexafluorophosphate | 148893-10-1 | DMU930 | Bide |
| 8 | Hydrogen chloride in 1,4-dioxane solution | 7647-01-0 | P2192569 | Titan |
| 9 | Polyphosphoric acid | 8017-16-1 | P1856081 | Titan |
| 10 | Diisobutylaluminum hydride (1.0 M) in n-hexane solution | 1191-15-7 | C15759865 | Macklin |
| 11 | Sodium hydroxide | 1310-73-2 | C2427250 | Aladdin |
| 12 | Methyl tert-butyl ether | 1634-04-4 | P2563275 | Titan |
| 13 | Oxalyl chloride | 79-37-8 | C14978996 | Macklin |
| 14 | Cuprous bromide | 7787-70-4 | C14302174 | Macklin |
| 15 | Cuprous iodide | 7681-65-4 | A2218355 | Aladdin |
| 16 | Anhydrous potassium carbonate, granular | 584-08-7 | P2857341 | Titan |
| 17 | 6-Hydroxypyridine-2-carboxylic acid | 19621-92-2 | D23040055 | Accela |
| 18 | 2,6-Dimethylaniline | 87-62-7 | P2954244 | Titan |
| 19 | Dimethyl sulfoxide | 67-68-5 | P3174042 | Titan |
| 20 | 1-tert-Butoxycarbonyl-1-methylhydrazine | 21075-83-2 | R23093769 | Accela |
| 21 | N1,N2-Bis(2,5-dimethyl-1H-pyrrol-1-yl)oxalamide | 289708-48-1 | | Titan |
| 22 | Hexadecyl trimethyl ammonium bromide | 57-09-0 | PIBURXA X | Energy |
| 23 | Ethyl pyruvate | 617-35-6 | P2432559 | Titan |
| 24 | 2-Bromopyridine-4-carbaldehyde | 118289-17-1 | FLX681 | Bide |
| 25 | n-Butyllithium (1.6 M/15%) in n-hexane solution | 109-72-8 | PG5URJD R | Energy |
| 26 | Dimethylformamide | 68-12-2 | K2321131 | Aladdin |
| 27 | Ethyl acrylate | 140-88-5 | J2121251 | Aladdin |
| 28 | (1R,2R)-1-Amino-2-(dimethylamino)cyclohexane | 320778-92-5 | P2832979 | Titan |
| 29 | Phenyl isothiocyanate | 103-72-0 | C16515779 | Macklin |
| 30 | Quinine | 130-95-0 | DGU980 | Bide |
| 31 | Quinindium | 56-54-2 | R23083839 | Accela |
| 32 | 3,5-Bis(trifluoromethyl)benzenesulfonyl chloride | 39234-86-1 | CNC497 | Bide |
| 33 | 3,5-Bis(trifluoromethyl)phenyl isothiocyanate | 23165-29-9 | DSV644 | Bide |
| 34 | 3-[[3,5-Bis(trifluoromethyl)phenyl]amino]-4-[[(1S,2S)-2-(dimethylamino)cyclohexyl]amino]-3-cyclobutene-1,2-dione | 1263205-96-4 | P2141437 | Titan |
| 35 | Novozym 435 | 9001-62-1 | | |

In Table 1, the materials with serial numbers 28-33 are raw materials for chiral catalysts. The starting materials and some catalysts are sourced from the Exploration Platform, Energy Chemical, and Wanghua Technology Platform, while some catalysts are synthesized in-house, with references: Highly enantioselective desymmetrizations of meso-anhydrides. Carsten Bolm. [J] Tetrahedron 2010, 66 6349. DOI: 10.1016/j.tet.2010.04.121; and Enantioselective Alcoholysis of meso-Glutaric Anhydrides Catalyzed by Cinchona-Based Sulfonamide Catalysts. Choong Eui Song. [J] Adv. Synth. Catal. 2010, 352, 2211. DOI: 10.1002/adsc.201000289.

The present invention provides a method for synthesizing an aryl-substituted chiral tetrahydropyran ring via desymmetrization, including the following steps:
a) alcoholyzing a compound of formula I through organic small-molecule catalysis or enzyme catalysis to obtain a compound of formula III with high chiral purity;
b) reacting the compound of formula III with a methyl metal reagent to obtain a compound of formula IV;
c) reducing a lactone in the compound of formula IV to obtain a compound of formula V;
d) subjecting a compound of formula V-A to a copper-catalyzed hydrazination reaction to obtain a compound of formula VI;
e) subjecting the compound of formula VI to acid-catalyzed Fisher cyclization with a pyruvic acid derivative to obtain a compound of formula VII;
or step d) being replaced with step f),
f) allowing a compound of formula V-B to be treated with a metal reagent and react with N,N-dimethylformamide to obtain a compound of formula VIII; and
g) subjecting the compound of formula VIII to cyclization with an acrylic acid derivative to obtain a compound of formula IX;
where the compound of formula I is: 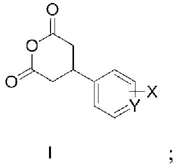
the compound of formula III is: 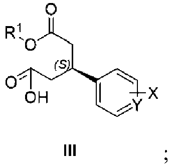
the compound of formula IV is: 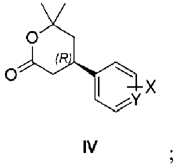
the compound of formula V is: 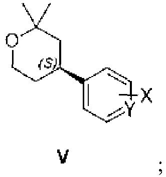
the compound of formula V-A is: 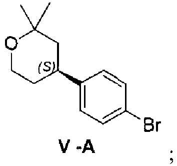
the compound of formula VI is: 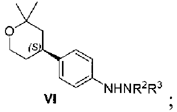
the compound of formula VII is: 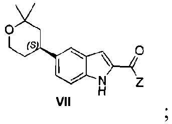
the compound of formula V-B is: 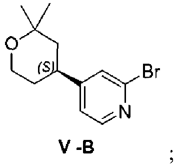
the compound of formula VIII is: 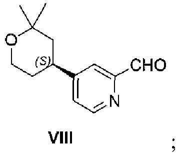 and
the compound of formula IX is: 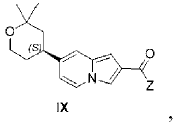
where in the formulas, X is halogen (Cl, Br, I), -OTf, -OMs, or -OTs; Y is C or N; R¹ is C1-C6 alkyl or benzyl; R² is H, Me, Et, iPr, or Bn; R³ is H or Boc; Z is -OR⁴; R⁴ is C1-C6 alkyl or -NR⁵R⁶; R⁵ is C1-C3 alkyl; and R⁶ is phenyl or substituted phenyl.

With reference to FIG. 2, the specific steps of the method for synthesizing an aryl-substituted chiral tetrahydropyran ring via desymmetrization in the present invention are as follows:
1. A compound of formula I is synthesized by referring to the literature "Pilot-Plant Preparation of 3,4-Dihydropyridin-2-one Derivatives, the Core Structures of P2X7 Receptor Antagonists" (By: Shu-Hai Zhao; et al., Organic Process Research & Development 2010, 14, 612-616).
2. The compound of formula I is dissolved in a solvent, and then methanol and a catalyst are added. The reaction is carried out at 0-50°C until completion, and after post-treatment, an intermediate of formula III is obtained;
alternatively, the compound of formula II is dissolved in a solvent, and an enzyme catalyst is added. The reaction is carried out in a buffer salt system until completion, and after post-treatment, an intermediate of formula III is obtained.

The compound of formula II is: 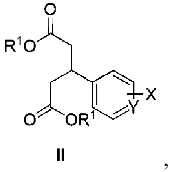 where in the formula, X is halogen (Cl, Br, I), -OTf, -OMs, or -OTs; Y is C or N; and R¹ is C1-C6 alkyl or benzyl.

In one embodiment of the present invention, a substrate for the alcoholysis reaction includes one of methanol, ethanol, benzyl alcohol, or other alkyl alcohols, and a quantity of the substrate as used in the alcoholysis reaction is 1.0-10.0 equivalents.

The catalyst used in the step is selected from a commercially available enzyme, such as Novozym 435, CAL-B, and small molecules including, but not limited to, those with the following structural characteristics:

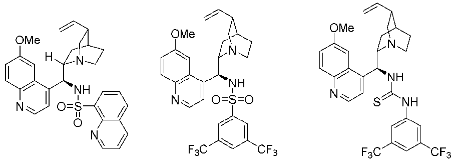

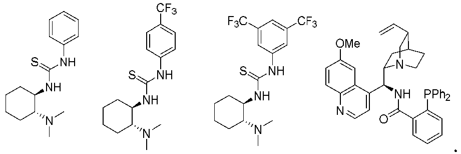

Preferably, the catalyst used in the step is selected from a commercially available enzyme, such as Novozym 435, amino lipase AYS, lipase PPL, etc.

3. The substance represented by the intermediate of formula III is dissolved in tetrahydrofuran, and a methyl metal reagent is added dropwise at low temperature. After completion of the reaction, a compound of formula IV is obtained through post-treatment.

In FIG. 2, Me-M represents MeLi, MeMgX (where X is Cl, Br, or I).

In one embodiment of the present invention, the methyl metal reagent includes one of methyllithium, methylmagnesium bromide, methylmagnesium chloride, or methylmagnesium iodide; and a quantity of the methyl metal reagent as used is 3.0-5.0 equivalents.

4. The substance of formula IV is dissolved in a solvent, a reducing reagent is added, and after post-treatment, a compound of formula V is obtained.

In one embodiment of the present invention, the reducing agent used for reducing the lactone includes DIBAL-H and Et₃SiH.

5. A substance of formula V-A is dissolved in a solvent, a substrate hydrazine, a copper catalyst, and a ligand are added. The reaction is carried out at 40-100°C under a nitrogen atmosphere until completion, and after post-treatment, a compound of formula VI is obtained.

In one embodiment of the present invention, the copper catalyst includes CuI or CuBr, and a quantity of the copper catalyst as used is 0.01-1.0 equivalents; and a ligand used in the reaction includes N-(2,6-dimethylphenyl)-6-hydroxypyridinecarboxamide or N1,N2-bis(2,5-dimethyl-1H-pyrrol-1-yl)oxalamide; and a substrate for the hydrazination reaction includes hydrazine hydrate, 1-tert-butoxycarbonyl-1-methylhydrazine, 1-tert-butoxycarbonyl-1-ethylhydrazine, or 1-tert-butoxycarbonyl-1-benzylhydrazine.

6. The substance of formula VI is dissolved in a solvent, a pyruvic acid derivative and an acid catalyst are added. The reaction is carried out at 40-100°C until completion, and after post-treatment, a compound of formula VII is obtained.

In one embodiment of the present invention, an acid used for the acid catalysis includes HCl, PPA, TsOH, or TfOH; and the pyruvic acid derivative includes ethyl pyruvate, methyl pyruvate, or N-methyl-2-oxo-N-phenylpropanamide.

7. A substance of formula V-B is dissolved in a solvent, a metal reagent is added dropwise at low temperature for lithiation, followed by a reaction with DMF (N,N-Dimethylformamide). After post-treatment, a compound of formula VIII is obtained.

In one embodiment of the present invention, the metal reagent includes n-butyllithium, sec-butyllithium, isopropylmagnesium chloride, or isopropylmagnesium bromide.

8. The substance of formula VIII is dissolved in a solvent, and an acrylic acid derivative is added, followed by a catalytic cyclization reaction. After post-treatment, a compound of formula IX is obtained.

In one embodiment of the present invention, the acrylic acid derivative includes ethyl acrylate, methyl acrylate, benzyl acrylate, or N-methyl-N-phenylacrylamide.

The following provides, through specific examples, the synthesis of ethyl (S)-5-(2,2-dimethyltetrahydro-2H-pyran-4-yl)-1H-indole-2-carboxylate (VII) and the synthesis of ethyl (S)-7-(2,2-dimethyltetrahydro-2H-pyran-4-yl)indolizine-2-carboxylate (IX).

### Synthesis of ethyl (S)-5-(2,2-dimethyltetrahydro-2H-pyran-4-yl)-1H-indole-2-carboxylate (VII):

### Example 1

### Synthesis of starting materials:

### Synthesis of 4-(4-bromophenyl)dihydro-2H-pyran-2,6(3H)-dione (I-A):

Under a nitrogen atmosphere, 285 g of p-bromobenzaldehyde, 1500 mL of ethanol, and 400 g of ethyl acetoacetate were sequentially added to a 5-L four-necked flask at room temperature. After purging with nitrogen, 20 g of piperidine was added, and the reaction was carried out at room temperature for 1 day. After the completion of the reaction as detected by LCMS (Liquid Chromatography-Mass Spectrometry), the mixture was directly filtered. The filter cake was washed by adding 550 mL of ethanol, suction-dried, and air-dried to obtain 460 g of diethyl 2,4-diacetyl-3-(4-bromophenyl)glutarate as an off-white solid (yield: 70.0%, purity: 93.9%). Next, 460 g of diethyl 2,4-diacetyl-3-(4-bromophenyl)glutarate and 1400 mL of ethanol were added to a 5-L four-necked flask at room temperature. 259.2 g of sodium hydroxide was dissolved in 1400 mL of water, and then added to the reaction mixture. The temperature was raised to 80°C, and the system gradually dissolved and became clear. The reaction was continued at 80°C for 2 hours. After the completion of the reaction as detected by LCMS, the mixture was cooled to room temperature. Most of the ethanol was removed by evaporation under reduced pressure in a 40°C water bath. Then the residue was diluted with 5000 mL of water, the solution was cooled with ice, and 594 mL of concentrated hydrochloric acid was added dropwise to adjust the pH to 3, resulting in the precipitation of a large amount of light yellow solid. The solid was filtered, and the filter cake was washed with 1000 mL of water, suction-dried, and air-dried. The sample was further dried at 45°C for 4 hours to obtain 278 g of 3-(4-bromophenyl)glutaric acid as a light yellow solid (yield: 90.6%, purity: 98.2%).

Under a nitrogen atmosphere, 1600 mL of acetyl chloride was added to a 5-L four-necked flask at room temperature, and 275 g of 3-(4-bromophenyl)glutaric acid was added portionwise. The mixture was then heated to 80°C, and the raw materials gradually dissolved (internal temperature: 58°C). The reaction was continued at 80°C for 2 hours. After the completion of the reaction as detected by LCMS with methanol derivatization, the mixture was cooled to room temperature. The solvent was removed by evaporation under reduced pressure in a 40°C water bath. The residue was triturated by adding 1000 mL of n-heptane, filtered, washed with 500 mL of n-heptane, and suction-dried. Then the solid was subjected to solvent removal by evaporation under reduced pressure in a 40°C water bath to finally obtain 212 g of an off-white solid (yield: 81.4%, purity: 97.4%).

The structural formula of the substance of formula I-A is:

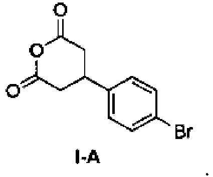

### Synthesis of dimethyl 3-(4-bromophenyl)glutarate (II-A):

Under a nitrogen atmosphere, 10 g of 3-(4-bromophenyl)glutaric acid, 100 mL of methanol, and 300 mg of concentrated sulfuric acid were sequentially added to a 250-mL three-necked flask. The mixture was refluxed at 70°C overnight. After the reaction, the mixture was cooled to room temperature, and the solvent was removed by evaporation under reduced pressure in a 40°C water bath. The crude residue was diluted with 200 mL of ethyl acetate, and washed with 50 mL of a semi-saturated aqueous sodium bicarbonate solution and saturated brine separately. The organic phase was collected, dried over anhydrous sodium sulfate, and filtered. The filtrate was subjected to solvent removal by evaporation under reduced pressure in a 40°C water bath to obtain 9 g of dimethyl 3-(4-bromophenyl)glutarate as a light brown solid (yield: 82%, purity: 96.5%).

The structural formula of the substance of formula II-A is: 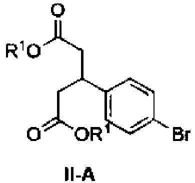 , where in the formula, R¹ is Me.

### Example 2

The structural formula of the substance of formula III-A is: 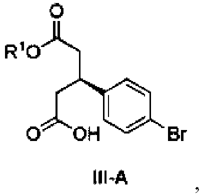 where in the formula, R¹ is Me.

### Step 1: synthesis of (S)-3-(4-bromophenyl)-5-methoxy-5-oxopentanoic acid (III-A) with reference to FIG. 2:

Under a nitrogen atmosphere, 2 g of 4-(4-bromophenyl)dihydro-2H-pyran-2,6(3H)-dione (I-A) prepared in Example 1, 200 mL of methyl tert-butyl ether, 2 g of methanol, and 0.04 g of 1-((1R,2R)-2-(dimethylamino)cyclohexyl)-3-(4-(trifluoromethyl)phenyl)thiourea were sequentially added to a 500-mL three-necked flask at room temperature. The reaction was carried out at room temperature for 1 day. After the completion of the reaction as detected by LCMS, the mixture was washed with 0.1 N hydrochloric acid and saturated brine. The upper organic phase was collected, dried over anhydrous sodium sulfate, and filtered. The filtrate was subjected to solvent removal by evaporation under reduced pressure, crystallized using an n-heptane/toluene system, and filtered. The resulting filtrate was collected and subjected to solvent removal to obtain 1.7 g of (S)-3-(4-bromophenyl)-5-methoxy-5-oxopentanoic acid (III-A) as an off-white solid (yield: 76.8%, purity: 99.5%, ee: 92.3%).

Alternatively, Step 1: synthesis of (S)-3-(4-bromophenyl)-5-methoxy-5-oxopentanoic acid (III-A) with reference to FIG. 2:

Under a nitrogen atmosphere, 2 g of 4-(4-bromophenyl)dihydro-2H-pyran-2,6(3H)-dione (I-A) prepared in Example 1, 200 mL of methyl tert-butyl ether, 2 g of methanol, and 0.04 g of 1-(3,5-bis(trifluoromethyl)phenyl)-3-((1R,2R)-2-(dimethylamino)cyclohexyl)thiourea were sequentially added to a 500-mL three-necked flask at room temperature. The reaction was carried out at room temperature for 1 day. After the completion of the reaction as detected by LCMS, the mixture was washed with 0.1 N hydrochloric acid and saturated brine. The upper organic phase was collected, dried over anhydrous sodium sulfate, and filtered. The filtrate was subjected to solvent removal by evaporation under reduced pressure, crystallized using an n-heptane/toluene system, and filtered. The resulting filtrate was collected and subjected to solvent removal to obtain 1.4 g of (S)-3-(4-bromophenyl)-5-methoxy-5-oxopentanoic acid (III-A) as an off-white solid (yield: 63.3%, purity: 99.1%, ee: 97.7%).

### Alternatively, Step 1: synthesis of (S)-3-(4-bromophenyl)-5-methoxy-5-oxopentanoic acid (III-A) with reference to FIG. 2:

Under a nitrogen atmosphere, 2 g of 4-(4-bromophenyl)dihydro-2H-pyran-2,6(3H)-dione (I-A) prepared in Example 1, 200 mL of methyl tert-butyl ether, 2 g of methanol, and 0.04 g of 1-(3,5-bis(trifluoromethyl)phenyl)-3-((S)-(6-methoxyquinolin-4-yl)((1S,2S,4S,5R)-5-vinylquinolin-2-yl)methyl)thiourea were sequentially added to a 500-mL three-necked flask at room temperature. The reaction was carried out at room temperature for 1 day. After the completion of the reaction as detected by LCMS, the mixture was washed with 0.1 N hydrochloric acid and saturated brine. The upper organic phase was collected, dried over anhydrous sodium sulfate, and filtered. The filtrate was subjected to solvent removal by evaporation under reduced pressure, crystallized using an n-heptane/toluene system, and filtered. The resulting filtrate was collected and subjected to solvent removal to obtain 1.6 g of (S)-3-(4-bromophenyl)-5-methoxy-5-oxopentanoic acid (III-A) as an off-white solid (yield: 71.4%, purity: 99.0%, ee: 92.5%).

### Alternatively, Step 1: synthesis of (S)-3-(4-bromophenyl)-5-methoxy-5-oxopentanoic acid (III-A) with reference to FIG. 2:

Under a nitrogen atmosphere, 2 g of 4-(4-bromophenyl)dihydro-2H-pyran-2,6(3H)-dione (I-A) prepared in Example 1, 200 mL of methyl tert-butyl ether, 2 g of methanol, and 0.04 g of N-((S)-(6-methoxyquinolin-4-yl)((1S,2S,4S,5R)-5-vinylquinolin-2-yl)methyl)-3,5-bis(trifluoromethyl)benzenesulfonamide were sequentially added to a 500-mL three-necked flask at room temperature. The reaction was carried out at room temperature for 1 day. After the completion of the reaction as detected by LCMS, the mixture was washed with 0.1 N hydrochloric acid and saturated brine. The upper organic phase was collected, dried over anhydrous sodium sulfate, and filtered. The filtrate was subjected to solvent removal by evaporation under reduced pressure, crystallized using an n-heptane/toluene system, and filtered. The resulting filtrate was collected and subjected to solvent removal to obtain 1.52 g of (S)-3-(4-bromophenyl)-5-methoxy-5-oxopentanoic acid (III-A) as an off-white solid (yield: 67.9%, purity: 98.6%, ee: 93.5%).

### Alternatively, Step 1: synthesis of (S)-3-(4-bromophenyl)-5-methoxy-5-oxopentanoic acid (III-A) with reference to FIG. 2:

Under a nitrogen atmosphere, 2 g of 4-(4-bromophenyl)dihydro-2H-pyran-2,6(3H)-dione (I-A) prepared in Example 1, 200 mL of methyl tert-butyl ether, 2 g of methanol, and 0.04 g of 1-((1R,2R)-2-(dimethylamino)cyclohexyl)-3-phenylthiourea were sequentially added to a 500-mL four-necked flask at room temperature. The reaction was carried out at room temperature for 1 day. After the completion of the reaction as detected by LCMS, the mixture was washed with 0.1 N hydrochloric acid and saturated brine. The upper organic phase was collected, dried over anhydrous sodium sulfate, and filtered. The filtrate was subjected to solvent removal by evaporation under reduced pressure, crystallized using an n-heptane/toluene system, and filtered. The resulting filtrate was collected and subjected to solvent removal to obtain 1.54 g of (S)-3-(4-bromophenyl)-5-methoxy-5-oxopentanoic acid (III-A) as an off-white solid (yield: 69.0%, purity: 98.5%, ee: 94.1%).

### Alternatively, Step 1: synthesis of (S)-3-(4-bromophenyl)-5-methoxy-5-oxopentanoic acid (III-A) with reference to FIG. 2:

Under a nitrogen atmosphere, 2 g of 4-(4-bromophenyl)dihydro-2H-pyran-2,6(3H)-dione (I-A) prepared in Example 1, 240 mL of methyl tert-butyl ether, 2.4 g of methanol, and 200 mg of Novozym 435 lipase were sequentially added to a 500-mL four-necked flask at room temperature. The reaction was carried out at room temperature for 5 days. After the completion of the reaction as detected by LCMS, the mixture was filtered and washed with 40 mL of methyl tert-butyl ether. The filtrate was washed with 0.1 N hydrochloric acid and saturated brine. The upper organic phase was collected, dried over anhydrous sodium sulfate, and filtered. The filtrate was subjected to solvent removal by evaporation under reduced pressure, crystallized using an n-heptane/toluene system, and filtered. The resulting filtrate was collected and subjected to solvent removal to obtain 1.14 g of (S)-3-(4-bromophenyl)-5-methoxy-5-oxopentanoic acid (III-A) as an off-white solid (yield: 51.0%, purity: 96%, ee: 98.5%).

### Alternatively, Step 1: synthesis of (S)-3-(4-bromophenyl)-5-methoxy-5-oxopentanoic acid (III-A) with reference to FIG. 2:

Under a nitrogen atmosphere, 2 g of dimethyl 3-(4-bromophenyl)glutarate (II-A) prepared in Example 1, 16 mL of a buffer solution (pH=7.2), and 200 mg of amino lipase AYS were sequentially added to a 100-mL three-necked flask. The mixture was reacted at room temperature overnight. After the completion of the reaction as detected by LCMS, the pH of the reaction mixture dropped to 5, and was adjusted to pH=2 with concentrated hydrochloric acid. The mixture was filtered and washed with 50 mL of ethyl acetate. The filtrate was extracted with ethyl acetate and washed with saturated brine. The upper organic phase was collected, dried over anhydrous sodium sulfate, and filtered. The filtrate was subjected to solvent removal by evaporation under reduced pressure, crystallized using an n-heptane/toluene system, and filtered. The resulting filtrate was collected and subjected to solvent removal to obtain 1.35 g of (S)-3-(4-bromophenyl)-5-methoxy-5-oxopentanoic acid (III-A) as an off-white solid (yield: 71.0%, purity: 95%, ee: 94.2%).

### Alternatively, Step 1: synthesis of (S)-3-(4-bromophenyl)-5-methoxy-5-oxopentanoic acid (III-A) with reference to FIG. 2:

Under a nitrogen atmosphere, 2 g of diethyl 3-(4-bromophenyl)glutarate (II-B) prepared in Example 1, 16 mL of a buffer solution (pH=7.2), and 200 mg of amino lipase AYS were sequentially added to a 100-mL three-necked flask. The mixture was reacted at room temperature overnight. After the completion of the reaction as detected by LCMS, the reaction pH dropped to 5, and was adjusted to pH=2 with concentrated hydrochloric acid. The mixture was filtered and washed with 50 mL of ethyl acetate. The filtrate was extracted with ethyl acetate and washed with saturated brine. The upper organic phase was collected, dried over anhydrous sodium sulfate, and filtered. The filtrate was subjected to solvent removal by evaporation under reduced pressure, crystallized using an n-heptane/toluene system, and filtered. The resulting filtrate was collected and subjected to solvent removal to obtain 1.14 g of (S)-3-(4-bromophenyl)-5-ethoxy-5-oxopentanoic acid (III-A) as an off-white solid (yield: 62.0%, purity: 96%, ee: 94.4%).

### Example 3

The structural formula of the substance of formula IV-A is:

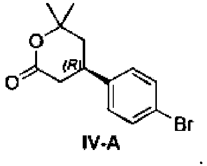

### Step 2: synthesis of (R)-4-(4-bromophenyl)-6,6-dimethyltetrahydro-2H-pyran-2-one (IV-A) with reference to FIG. 2:

Under a nitrogen atmosphere, 10 g of (S)-3-(4-bromophenyl)-5-methoxy-5-oxopentanoic acid (III-A) prepared in Example 2 and 100 mL of tetrahydrofuran were sequentially added to a 1000-mL three-necked flask. The mixture was cooled to -30°C, and 44.5 mL of methylmagnesium chloride (3 mol/L in THF (Tetrahydrofuran)) was added dropwise, maintaining the temperature no more than -10°C. After the dropwise addition, the reaction was carried out at 0°C for 2 hours. After the completion of the reaction as detected by LCMS, the reaction mixture was quenched by pouring into 150 mL of an ice-cold 1 N hydrochloric acid solution, extracted with ethyl acetate, and washed with saturated brine. The organic phase was collected, dried over anhydrous sodium sulfate, and filtered. The filtrate was subjected to solvent removal by rotary evaporation under reduced pressure. The crude product was triturated with ethyl acetate and n-heptane and filtered. The filter cake was suction-dried to obtain 10.4 g of (R)-4-(4-bromophenyl)-6,6-dimethyltetrahydro-2H-pyran-2-one (IV-A) as a white solid (yield: 75.3%, purity: 98.7%, ee: 96.8%).

### Alternatively, Step 2: synthesis of (R)-4-(4-bromophenyl)-6,6-dimethyltetrahydro-2H-pyran-2-one (IV-A) with reference to FIG. 2:

Under a nitrogen atmosphere, 10 g of (S)-3-(4-bromophenyl)-5-methoxy-5-oxopentanoic acid (III-A) prepared in Example 2 and 100 mL of tetrahydrofuran were sequentially added to a 1000-mL three-necked flask. The mixture was cooled to -40°C, and 45 mL of methylmagnesium chloride (3 mol/L in THF) was added dropwise, maintaining the temperature no more than -10°C. After the dropwise addition, the reaction was carried out at 10°C for 2 hours. After the completion of the reaction as detected by LCMS, the reaction mixture was quenched by pouring into 150 mL of an ice-cold 1 N hydrochloric acid solution, extracted with ethyl acetate, and washed with saturated brine. The organic phase was collected, dried over anhydrous sodium sulfate, and filtered. The filtrate was subjected to solvent removal by rotary evaporation under reduced pressure. The crude product was triturated with ethyl acetate and n-heptane and filtered. The filter cake was suction-dried to obtain 11.5 g of (R)-4-(4-bromophenyl)-6,6-dimethyltetrahydro-2H-pyran-2-one (IV-A) as a white solid (yield: 83.3%, purity: 97.8%, ee: 94.8%).

### Alternatively, Step 2: synthesis of (R)-4-(4-bromophenyl)-6,6-dimethyltetrahydro-2H-pyran-2-one (IV-A) with reference to FIG. 2:

Under a nitrogen atmosphere, 10 g of (S)-3-(4-bromophenyl)-5-methoxy-5-oxopentanoic acid (III-A) prepared in Example 2 and 100 mL of tetrahydrofuran were sequentially added to a 1000-mL three-necked flask. The mixture was cooled to -40°C, and 130 mL of methyllithium (1 mol/L in 2-MeTHF) was added dropwise, maintaining the temperature no more than -10°C. After the dropwise addition, the reaction was carried out at 0°C for 2 hours. After the completion of the reaction as detected by LCMS, the reaction mixture was quenched by pouring into 150 mL of an ice-cold 1 N hydrochloric acid solution, extracted with ethyl acetate, and washed with saturated brine. The organic phase was collected, dried over anhydrous sodium sulfate, and filtered. The filtrate was subjected to solvent removal by rotary evaporation under reduced pressure. The crude product was triturated with ethyl acetate and n-heptane and filtered. The filter cake was suction-dried to obtain 12.3 g of (R)-4-(4-bromophenyl)-6,6-dimethyltetrahydro-2H-pyran-2-one (IV-A) as a white solid (yield: 89.1%, purity: 95.8%, ee: 91.8%).

### Example 4

The structural formula of the substance of formula V-A is:

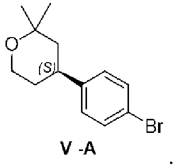

### Step 3: synthesis of (S)-4-(4-bromophenyl)-2,2-dimethyltetrahydro-2H-pyran (V-A) with reference to FIG. 2:

In a 250-mL three-necked flask, 10 g of (R)-4-(4-bromophenyl)-6,6-dimethyltetrahydro-2H-pyran-2-one (IV-A) prepared in Example 3 and 100 mL of dichloromethane were sequentially added. The mixture was cooled to -70°C in a dry ice/ethanol bath, and 50 mL of a DIBAL-H solution (1 mol/L in hexane) was added dropwise, maintaining the temperature no more than -65°C. After the dropwise addition, the reaction was kept at -70°C for 1 hour. After the completion of the reaction as detected by LCMS, the reaction mixture was quenched by pouring into 300 mL of a 10% wt aqueous sodium potassium tartrate solution, and stirred for 1 hour until clear and separated into layers. The mixture was extracted with DCM (Dichloromethane), washed with saturated brine, dried over anhydrous sodium sulfate, filtered, and subjected to solvent removal by evaporation under reduced pressure to obtain 9.6 g of (4R)-4-(4-bromophenyl)-6,6-dimethyltetrahydro-2H-pyran-2-ol as a white solid. The solid was added to a 250-mL three-necked flask with 100 mL of DCM and 11 g of triethylsilane, and cooled to 0°C in an ice-water bath. 5.7 g of boron trifluoride diethyl etherate was added dropwise, followed by a reaction at 0°C for 2 hours. After the completion of the reaction as detected by LCMS, the reaction mixture was quenched by pouring into 100 mL of an ice-cold semi-saturated aqueous sodium bicarbonate solution, and stirred until no bubbles formed. The mixture was extracted with DCM, washed with saturated brine, dried over anhydrous sodium sulfate, and filtered. The filtrate was subjected to solvent removal by evaporation under reduced pressure to obtain a colorless oil. The oil was crystallized from n-heptane/ethyl acetate to obtain 8.2 g of (S)-4-(4-bromophenyl)-2,2-dimethyltetrahydro-2H-pyran (V-A) (yield: 94.2%, purity: 93.0%).

### Example 5

The structural formula of the substance of formula VI is 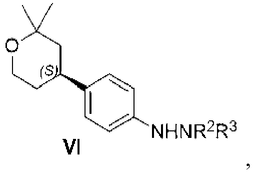 where in the formula, R²=H, and R³=H.

### Step 4: synthesis of (S)-(4-(2,2-dimethyltetrahydro-2H-pyran-4-yl)phenyl)hydrazine hydrochloride (VI) with reference to FIG. 2:

In a 100-mL three-necked flask with magnetic stirring under a nitrogen atmosphere, 18 mg of cuprous iodide, 41 mg of N1,N2-bis(2,5-dimethyl-1H-pyrrol-1-yl)oxalamide, 95 mg of potassium phosphate, and 41 mg of cetyltrimethylammonium bromide were sequentially added. Then, 1 g of (S)-4-(4-bromophenyl)-2,2-dimethyltetrahydro-2H-pyran (V-A) prepared in Example 4 and 0.25 mL of water were added. The system was purged with nitrogen 3 times, and reacted at 80°C for 15 minutes. After the system was cooled to room temperature, 853 mg of potassium phosphate and 450 mg of 85% wt hydrazine hydrate were added. The mixture was purged with nitrogen 3 times, and then reacted at 80°C overnight. After being cooled to room temperature, the mixture was diluted by adding 20 mL of dichloromethane and filtered. The filter cake was washed with 20 mL of dichloromethane. The filtrate was washed with 20 mL of water followed by semi-saturated brine once. The organic phase was collected, adjusted to pH=3 by adding concentrated hydrochloric acid dropwise to precipitate a large amount of solid, and filtered. The filter cake was washed with 10 mL of dichloromethane, suction-dried, and subjected to solvent removal by evaporation under reduced pressure in a 40°C water bath to obtain 670 mg of (S)-(4-(2,2-dimethyltetrahydro-2H-pyran-4-yl)phenyl)hydrazine hydrochloride (VI) as an off-white solid (yield: 70.1%, purity: 96.5%).

### Alternatively, Step 4: synthesis of (S)-(4-(2,2-dimethyltetrahydro-2H-pyran-4-yl)phenyl)hydrazine hydrochloride (VI) with reference to FIG. 2:

In a 100-mL three-necked flask with magnetic stirring under a nitrogen atmosphere, 62 mg of cuprous iodide, 143 mg of N1,N2-bis(2,5-dimethyl-1H-pyrrol-1-yl)oxalamide, 332 mg of potassium phosphate, and 143 mg of cetyltrimethylammonium bromide were sequentially added. Then, 3.5 g of (S)-4-(4-bromophenyl)-2,2-dimethyltetrahydro-2H-pyran (V-A) prepared in Example 4 and 0.9 mL of water were added. The system was purged with nitrogen 3 times, and reacted at 80°C for 15 minutes. After the system was cooled to room temperature, 2987 mg of potassium phosphate and 1.54 mg of 85% wt hydrazine hydrate were added. The mixture was purged with nitrogen 3 times, and then reacted at 80°C overnight. After being cooled to room temperature, the mixture was diluted by adding 60 mL of dichloromethane and filtered. The filter cake was washed with 60 mL of dichloromethane. The filtrate was washed with 30 mL of water followed by 30 mL of semi-saturated brine once. The organic phase was collected, adjusted to pH=3 by adding concentrated hydrochloric acid dropwise to precipitate a large amount of solid, and filtered. The filter cake was washed with 30 mL of dichloromethane, suction-dried, and subjected to solvent removal by evaporation under reduced pressure in a 40°C water bath to obtain 2.78 g of (S)-(4-(2,2-dimethyltetrahydro-2H-pyran-4-yl)phenyl)hydrazine hydrochloride (VI) as an off-white solid (yield: 83.2%, purity: 96.5%).

The structural formula of the substance of formula VI is: 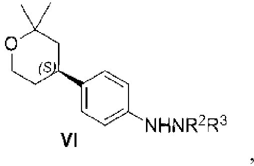 where R²=Me, and R³=Boc.

### Step 4: synthesis of tert-butyl (S)-2-(4-(2,2-dimethyltetrahydro-2H-pyran-4-yl)phenyl)-1-methylhydrazine-1-carboxylate (VI) with reference to FIG. 2:

In a 100-mL three-necked flask with magnetic stirring under a nitrogen atmosphere, 57 mg of cuprous iodide, 61 mg of N1,N2-bis(2,5-dimethyl- l H-pyrrol-1-yl)oxalamide, and 772 mg of potassium carbonate were sequentially added. The system was purged with nitrogen 3 times. Then, 1 g of (S)-4-(4-bromophenyl)-2,2-dimethyltetrahydro-2H-pyran (V-A) prepared in Example 4, 0.68 g of tert-butyl 1-methylhydrazine-1-carboxylate, and 20 mL (10V) of dimethyl sulfoxide were added. After 3 nitrogen purges, the mixture was reacted at 80°C overnight. After most of the raw materials were converted to the product as detected by LCMS, the mixture was cooled to room temperature and filtered. The filter cake was washed with 40 mL of ethyl acetate, and the filtrate was quenched by pouring into 60 mL of ice water. The mixture was extracted with ethyl acetate and washed with saturated brine. The organic phase was collected, dried over anhydrous sodium sulfate, and filtered. The filtrate was subjected to solvent removal by evaporation under reduced pressure in a 40°C water bath. The crude product was purified by wet column chromatography on silica gel (heptane/EA = 6:1) to obtain 475 mg of tert-butyl (S)-2-(4-(2,2-dimethyltetrahydro-2H-pyran-4-yl)phenyl)-1-methylhydrazine-1-carboxylate (VI-B) as a colorless oil (yield: 19.1%, purity: 95%).

### Step 4: synthesis of tert-butyl (S)-2-(4-(2,2-dimethyltetrahydro-2H-pyran-4-yl)phenyl)-1-methylhydrazine-1-carboxylate (VI) with reference to FIG. 2:

In a 250-mL three-necked flask with magnetic stirring under a nitrogen atmosphere, 50 mg of cuprous bromide, 108 mg of N-(2,6-dimethylphenyl)-6-hydroxypyridineamide, and 1.54 g of potassium carbonate were sequentially added. The system was purged with nitrogen 3 times. Then, 2 g of (S)-4-(4-bromophenyl)-2,2-dimethyltetrahydro-2H-pyran (V-A) prepared in Example 4, 1.36 g of tert-butyl 1-methylhydrazine-1-carboxylate, and 20 mL of dimethyl sulfoxide were added. After 3 nitrogen purges, the mixture was reacted at 80°C overnight. After the completion of the reaction as detected by LCMS, the mixture was cooled to room temperature and filtered. The filter cake was washed with 40 mL of ethyl acetate, and the filtrate was quenched by pouring into 60 mL of ice water. The mixture was extracted with ethyl acetate and washed with saturated brine. The organic phase was collected, dried over anhydrous sodium sulfate, and filtered. The filtrate was subjected to solvent removal by evaporation under reduced pressure in a 40°C water bath. The crude product was purified by wet column chromatography on silica gel (heptane/EA = 6:1) to obtain 2.0 g of tert-butyl (S)-2-(4-(2,2-dimethyltetrahydro-2H-pyran-4-yl)phenyl)-1-methylhydrazine-1-carboxylate (VI-B) as a colorless oil (yield: 80.3%, purity: 96%).

### Example 6

The structural formula of the substance of formula VII is: 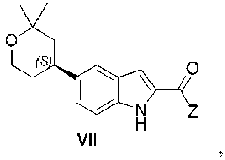 where in the formula, Z is OEt.

### Step 5: synthesis of ethyl (S)-5-(2,2-dimethyltetrahydro-2H-pyran-4-yl)-1H-indole-2-carboxylate (VII) with reference to FIG. 2:

In a 250-mL three-necked flask, 20 mL of ethanol was mixed with 4 g of concentrated sulfuric acid. After the mixture was cooled to room temperature, 2 g of (S)-(4-(2,2-dimethyltetrahydro-2H-pyran-4-yl)phenyl)hydrazine (VI) prepared in Example 5 and 760 mg of ethyl pyruvate were added. Then the mixture was reacted at 80°C for 4 hours. After the completion of the reaction as detected by LCMS, the mixture was cooled to room temperature. Most of the ethanol was removed by evaporation under reduced pressure in a 40°C water bath, and the residue was quenched by pouring into 50 mL of water. The mixture was extracted with ethyl acetate and washed with saturated brine. The organic phase was collected, dried over anhydrous sodium sulfate, and filtered. The filtrate was concentrated under reduced pressure and purified by silica gel column chromatography to obtain 820 mg of ethyl (S)-5-(2,2-dimethyltetrahydro-2H-pyran-4-yl)-1H-indole-2-carboxylate (VII) (yield: 45.4%, purity: 97.2%, ee: 95.7%).

### Alternatively, Step 5: synthesis of ethyl (S)-5-(2,2-dimethyltetrahydro-2H-pyran-4-yl)-1H-indole-2-carboxylate (VII) with reference to FIG. 2:

In a 250-mL three-necked flask, 20 mL of toluene was mixed with 5 g of polyphosphoric acid. Then, 2 g of (S)-(4-(2,2-dimethyltetrahydro-2H-pyran-4-yl)phenyl)hydrazine (VI) prepared in Example 5 and 750 mg of ethyl pyruvate were added. Then the mixture was reacted at 100°C for 4 hours. After the completion of the reaction as detected by LCMS, the mixture was cooled to room temperature, quenched by pouring into 50 mL of water, extracted with toluene, washed with saturated brine, concentrated under reduced pressure, and purified by silica gel column chromatography to obtain 870 mg of ethyl (S)-5-(2,2-dimethyltetrahydro-2H-pyran-4-yl)-1H-indole-2-carboxylate (VII) (yield: 48.2%, purity: 97.2%, ee: 96.9%).

### Alternatively, Step 5: synthesis of ethyl (S)-5-(2,2-dimethyltetrahydro-2H-pyran-4-yl)-1H-indole-2-carboxylate (VII) with reference to FIG. 2:

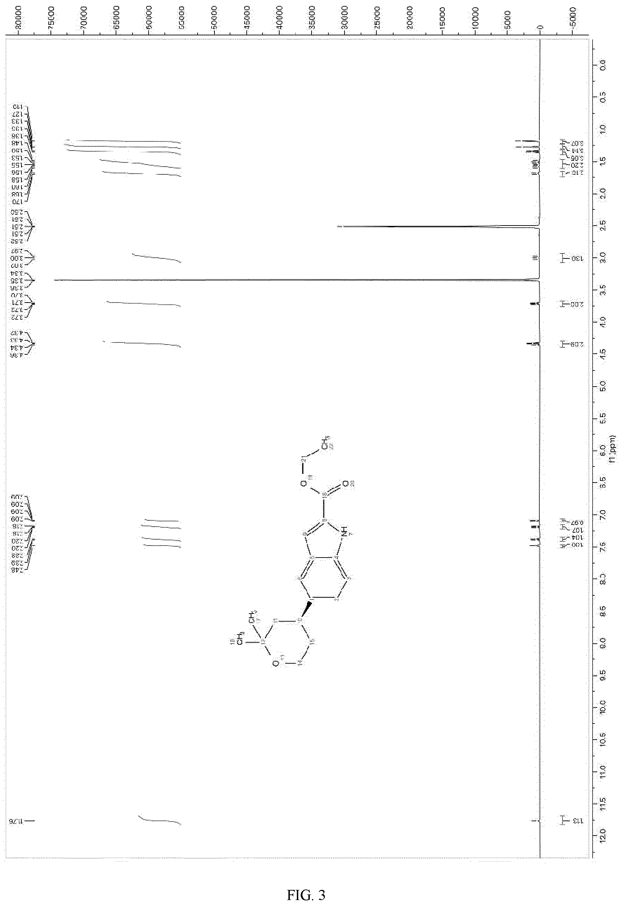

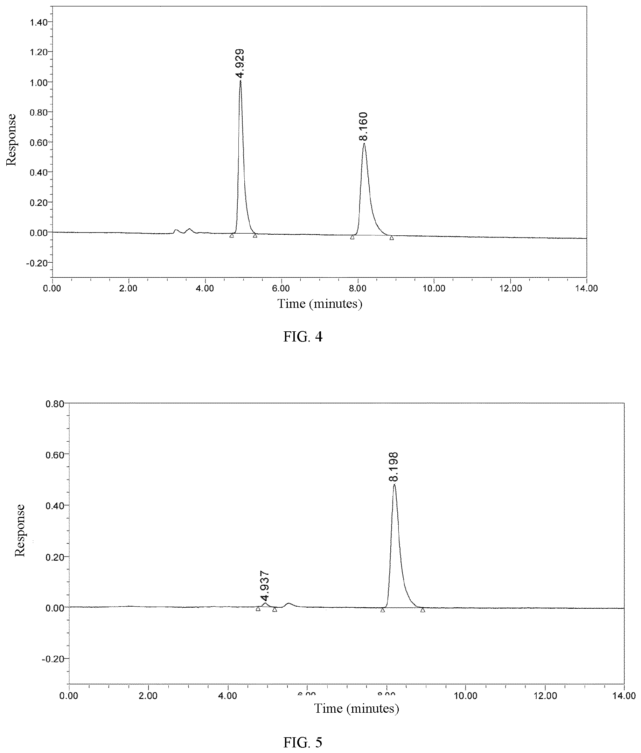

In a 250-mL three-necked flask, 20 mL of ethanol was mixed with 4 g of concentrated sulfuric acid. After the mixture was cooled to room temperature, 2 g of tert-butyl (S)-2-(4-(2,2-dimethyltetrahydro-2H-pyran-4-yl)phenyl)-1-methylhydrazine-1-carboxylate (VI-B) prepared in Example 5 and 760 mg of ethyl pyruvate were added. Then the mixture was reacted at 80°C for 4 hours. After the completion of the reaction as detected by LCMS, the mixture was cooled to room temperature. Most of the ethanol was removed by evaporation under reduced pressure in a 40°C water bath, and the residue was quenched by pouring into 50 mL of water. The mixture was extracted with ethyl acetate and washed with saturated brine. The organic phase was collected, dried over anhydrous sodium sulfate, and filtered. The filtrate was concentrated under reduced pressure and purified by silica gel column chromatography to obtain 980 mg of ethyl (S)-5-(2,2-dimethyltetrahydro-2H-pyran-4-yl)-1H-indole-2-carboxylate (VII) (yield: 54.4%, purity: 95.2%, ee: 94.7%). The nuclear magnetic resonance spectrum of the prepared (S)-ethyl 5-(2,2-dimethyltetrahydro-2H-pyran-4-yl)-1H-indole-2-carboxylate (VII) is shown in FIG. 3. The chiral chromatogram of the racemate is illustrated in FIG. 4 and Table 2, and the chiral chromatogram of the single chiral target is illustrated in FIG. 5 and Table 3.

**Table 2**

| | Retention time | Peak area | Peak area ratio | Peak height |
|---|---|---|---|---|
| 1 | 4.929 | 9582978 | 49.44 | 1017767 |
| 2 | 8.160 | 9799399 | 50.56 | 611174 |

**Table 3**

| | Retention time | Peak area | Peak area ratio | Peak height |
|---|---|---|---|---|
| 1 | 4.937 | 120780 | 1.55 | 14758 |
| 2 | 8.198 | 7678013 | 98.45 | 483093 |

### Alternatively, Step 5: synthesis of ethyl (S)-5-(2,2-dimethyltetrahydro-2H-pyran-4-yl)-1H-indole-2-carboxylate (VII) with reference to FIG. 2:

In a 250-mL three-necked flask with magnetic stirring under a nitrogen atmosphere, 2 g of tert-butyl (S)-2-(4-(2,2-dimethyltetrahydro-2H-pyran-4-yl)phenyl)-1-methylhydrazine-1-carboxylate (VI-B) prepared in Example 5, 765 mg of ethyl pyruvate, and 20 mL of ethanol were sequentially added. 1 g of trimethylchlorosilane was added dropwise at room temperature. Then the mixture was reacted at 80°C for 4 hours. After the completion of the reaction as detected by LCMS, the mixture was cooled to room temperature. Most of the ethanol was removed by evaporation under reduced pressure in a 40°C water bath, and the residue was quenched by pouring into 50 mL of water. The mixture was extracted with ethyl acetate and washed with saturated brine. The organic phase was collected, dried over anhydrous sodium sulfate, and filtered. The filtrate was concentrated under reduced pressure, and crystallized from n-heptane/ethyl acetate to obtain 732 mg of ethyl (S)-5-(2,2-dimethyltetrahydro-2H-pyran-4-yl)-1H-indole-2-carboxylate (VII) as an off-white solid (yield: 40.6%, purity: 96.27%, ee: 93.9%).

### Synthesis of ethyl (S)-7-(2,2-dimethyltetrahydro-2H-pyran-4-yl)indolizine-2-carboxylate (IX):

### Example 7

### Synthesis of starting materials:

### Synthesis of 4-(2-bromopyridin-4-yl)dihydro-2H-pyran-2,6(3H)-dione (I-B):

Under a nitrogen atmosphere, 200 g of 2-bromoisonicotinaldehyde, 1000 mL of ethanol, and 421.6 g of ethyl acetoacetate were sequentially added to a 5-L four-necked flask with mechanical stirring at room temperature. After purging with nitrogen, 13.8 g of piperidine was added, and the reaction was carried out at room temperature for 1 day. After the completion of the reaction as detected by LCMS, the mixture was filtered directly. The filter cake was washed with 400 mL of ethanol, suction-dried, and air-dried to obtain 365 g of diethyl 2,4-diacetyl-3-(2-bromopyridin-4-yl)glutarate as an off-white solid (yield: 79.1%, purity: 96%). Next, 365 g of diethyl 2,4-diacetyl-3-(2-bromopyridin-4-yl)glutarate and 1095 mL of ethanol were added to a 5-L four-necked flask at room temperature. 205.1 g of sodium hydroxide was dissolved in 1095 mL of water, cooled to room temperature, and then added to the reaction mixture. The temperature was raised to 40°C, and the system gradually dissolved and became clear. The reaction was continued at 40°C for 2 hours. After the completion of the reaction as detected by LCMS, the mixture was cooled to room temperature. Most of the ethanol was removed by evaporation under reduced pressure in a 40°C water bath. Then the residue was diluted with 1825 mL of water, the solution was cooled with ice, and 448 mL of concentrated hydrochloric acid was added dropwise to adjust the pH to 3, resulting in the precipitation of a large amount of off-white solid. The solid was filtered, and the filter cake was washed with 1000 mL of water, suction-dried, and air-dried. The sample was further dried at 45°C for 4 hours to obtain 182.5 g of 3-(2-bromopyridin-4-yl)glutaric acid as an off-white solid (yield: 74.4%, purity: 96%).

Under a nitrogen atmosphere, 180 g of 3-(2-bromopyridin-4-yl)glutaric acid, 1800 mL of toluene, and 191.2 g of acetic anhydride were added portionwise to a 5-L four-necked flask at room temperature. The mixture was then heated to 110°C, and the raw materials gradually dissolved. The reaction was continued at 110°C for 5 hours. After the completion of the reaction as detected by LCMS with methanol derivatization, the mixture was cooled to room temperature. The solvent was removed by evaporation under reduced pressure in a 40°C water bath. The residue was triturated by adding 1000 mL of n-heptane, filtered, washed with 500 mL of n-heptane, and suction-dried. Then the solid was subjected to solvent removal by evaporation under reduced pressure in a 40°C water bath to finally obtain 126.5 g of 4-(2-bromopyridin-4-yl)dihydro-2H-pyran-2,6(3H)-dione (I-B) as a light yellow solid (yield: 75.0%, purity: 94%).

The structural formula of the substance of formula I-B is:

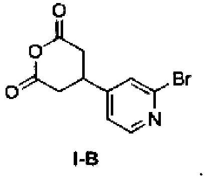

### Example 8

The structural formula of the substance of formula III-B is 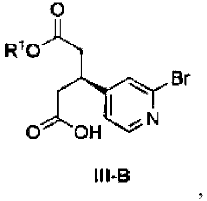 where in the formula, R¹ is Me.

### Step 1: synthesis of (S)-3-(2-bromopyridin-4-yl)-5-methoxy-5-oxopentanoic acid (III-B) with reference to FIG. 2:

Under a nitrogen atmosphere, 2 g of 4-(2-bromopyridin-4-yl)dihydro-2H-pyran-2,6(3H)-dione (I-B) prepared in Example 7, 200 mL of methyl tert-butyl ether, 2.4 g of methanol, and 0.04 g of 1-((1R,2R)-2-(dimethylamino)cyclohexyl)-3-phenylthiourea were sequentially added to a 500-mL four-necked flask at room temperature. The reaction was carried out at room temperature for 1 day. After the completion of the reaction as detected by LCMS, the mixture was washed with 0.1 N hydrochloric acid and saturated brine. The upper organic phase was collected, dried over anhydrous sodium sulfate, and filtered. The filtrate was subjected to solvent removal by evaporation under reduced pressure, crystallized using an n-heptane/toluene system, and filtered. The resulting filtrate was collected and subjected to solvent removal to obtain 1.45 g of (S)-3-(2-bromopyridin-4-yl)-5-methoxy-5-oxopentanoic acid (III-B) as an off-white solid (yield: 65.0%, purity: 99.5%, ee: 94.5%).

### Alternatively, Step 1: synthesis of (S)-3-(2-bromopyridin-4-yl)-5-methoxy-5-oxopentanoic acid (III-B) with reference to FIG. 2:

Under a nitrogen atmosphere, 2 g of 4-(2-bromopyridin-4-yl)dihydro-2H-pyran-2,6(3H)-dione (I-B) prepared in Example 7, 200 mL of methyl tert-butyl ether, 2.4 g of methanol, and 0.04 g of 1-(3,5-bis(trifluoromethyl)phenyl)-3-((S)-(6-methoxyquinolin-4-yl)((1S,2S,4S,5R)-5-vinylquinolin-2-yl)methyl)thiourea were sequentially added to a 500-mL four-necked flask at room temperature. The reaction was carried out at room temperature for 1 day. After the completion of the reaction as detected by LCMS, the mixture was washed with 0.1 N hydrochloric acid and saturated brine. The upper organic phase was collected, dried over anhydrous sodium sulfate, and filtered. The filtrate was subjected to solvent removal by evaporation under reduced pressure, crystallized using an n-heptane/toluene system, and filtered. The resulting filtrate was collected and subjected to solvent removal to obtain 1.15 g of (S)-3-(2-bromopyridin-4-yl)-5-methoxy-5-oxopentanoic acid (III-B) as an off-white solid (yield: 51.5%, purity: 98.5%, ee: 92.5%).

### Example 9

The structural formula of the substance of formula IV-B is:

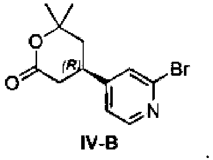

### Step 2: synthesis of (R)-4-(2-bromopyridin-4-yl)-6,6-dimethyltetrahydro-2H-pyran-2-one (IV-B) with reference to FIG. 2:

Under a nitrogen atmosphere, 10 g of (S)-3-(2-bromopyridin-4-yl)-5-methoxy-5-oxopentanoic acid (III-B) prepared in Example 8 and 100 mL of tetrahydrofuran were sequentially added to a 1000-mL three-necked flask. The mixture was cooled to -40°C, and 38 mL of methylmagnesium chloride (3 mol/L in THF) was added dropwise, maintaining the temperature no more than -10°C. After the dropwise addition, the reaction was carried out at room temperature for 2 hours. After the completion of the reaction as detected by LCMS, the reaction mixture was quenched by pouring into 150 mL of an ice-cold 1 N hydrochloric acid solution, extracted with ethyl acetate, and washed with saturated brine. The organic phase was collected, dried over anhydrous sodium sulfate, and filtered. The filtrate was subjected to solvent removal by evaporation under reduced pressure in a 40°C water bath. The crude product was triturated with ethyl acetate and n-heptane and filtered. The filter cake was suction-dried to obtain 6.8 g of (R)-4-(2-bromopyridin-4-yl)-6,6-dimethyltetrahydro-2H-pyran-2-one (IV-B) as a white solid (yield: 73.1%, purity: 98.7%, ee: 96.2%).

### Example 10

The structural formula of the substance of formula V-B is:

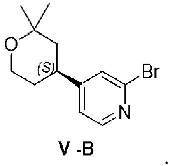

### Step 3: synthesis of (S)-2-bromo-4-(2,2-dimethyltetrahydro-2H-pyran-4-yl)pyridine (V-B) with reference to FIG. 2:

In a 250-mL three-necked flask, 10 g of (R)-4-(2-bromopyridin-4-yl)-6,6-dimethyltetrahydro-2H-pyran-2-one (IV-B) prepared in Example 9 and 100 mL of dichloromethane were sequentially added. The mixture was cooled to -70°C in a dry ice/ethanol bath, and 48 mL of a DIBAL-H solution (1 mol/L in hexane) was added dropwise, maintaining the temperature no more than -65°C. After the dropwise addition, the reaction was kept at -70°C for 1 hour. After the completion of the reaction as detected by LCMS, the reaction mixture was quenched by pouring into 300 mL of a 10% wt aqueous sodium potassium tartrate solution, and stirred for 1 hour until clear and separated into layers. The mixture was extracted with DCM, washed with saturated brine, dried over anhydrous sodium sulfate, filtered, and subjected to solvent removal by evaporation under reduced pressure to obtain 10 g of (4R)-4-(2-bromopyridin-4-yl)-6,6-dimethyltetrahydro-2H-pyran-2-ol as a white solid. The solid was added to a 250-mL three-necked flask with 100 mL of DCM and 11 g of triethylsilane, and cooled to 0°C in an ice-water bath. 5.7 g of boron trifluoride diethyl etherate was added dropwise, followed by a reaction at 0°C for 2 hours. After the completion of the reaction as detected by LCMS, the reaction mixture was quenched by pouring into 100 mL of an ice-cold semi-saturated aqueous sodium bicarbonate solution, and stirred until no bubbles formed. The mixture was extracted with DCM, washed with saturated brine, dried over anhydrous sodium sulfate, and filtered. The filtrate was subjected to solvent removal by evaporation under reduced pressure in a 40°C water bath to obtain a colorless oil. The oil was crystallized from n-heptane/ethyl acetate to obtain 8.6 g of (S)-2-bromo-4-(2,2-dimethyltetrahydro-2H-pyran-4-yl)pyridine (V-B) (yield: 90.5%, purity: 96.0%).

### Example 11

The substance of formula VIII is:

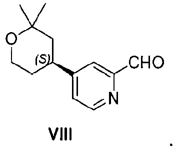

### Step 4: synthesis of (S)-4-(2,2-dimethyltetrahydro-2H-pyran-4-yl)pyridinecarbaldehyde (VIII) with reference to FIG. 2:

In a 500-mL three-necked flask, 10 g of (S)-2-bromo-4-(2,2-dimethyltetrahydro-2H-pyran-4-yl)pyridine (V-B) prepared in Example 10 and 100 mL of tetrahydrofuran were sequentially added. The mixture was cooled to -70°C in a dry ice/ethanol bath, and 55.7 mL of an n-butyllithium solution (1 mol/L in THF) was added dropwise. After the dropwise addition, the reaction was kept at -70°C for half an hour. Then, 27.1 g of DMF was added dropwise, maintaining the temperature no more than -65°C. After the dropwise addition, the temperature was slowly raised to 0°C, and the reaction was carried out for 1 hour. After the completion of the reaction as detected by LCMS, the reaction mixture was quenched by pouring into 100 mL of an ice-cold saturated aqueous ammonium chloride solution, extracted with ethyl acetate, washed with saturated brine, dried over anhydrous sodium sulfate, and filtered. The filtrate was subjected to solvent removal by evaporation under reduced pressure in a 40°C water bath and crystallized from n-heptane/ethyl acetate to obtain 5.7 g of (S)-4-(2,2-dimethyltetrahydro-2H-pyran-4-yl)pyridinecarbaldehyde (VIII) (yield: 70.0%, purity: 94.3%).

### Example 12

The substance of formula IX is: 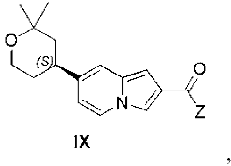 where in the formula, Z is OEt.

### Step 5: synthesis of ethyl (S)-7-(2,2-dimethyltetrahydro-2H-pyran-4-yl)indolizine-2-carboxylate (IX) with reference to FIG. 2:

In a 100-mL three-necked flask with magnetic stirring under a nitrogen atmosphere, 5 g of (S)-4-(2,2-dimethyltetrahydro-2H-pyran-4-yl)pyridinecarbaldehyde (VIII) prepared in Example 11, 4.6 g of ethyl acrylate, and 1.7 g of DABCO were sequentially added. The mixture was reacted at room temperature for 12 hours. After the completion of the reaction as detected by LCMS, ethyl acrylate was removed under reduced pressure in a 40°C water bath. The crude residue was dissolved in 100 mL of ethyl acetate, and washed with water and saturated brine separately. The organic phase was collected, dried over anhydrous sodium sulfate, filtered, and subjected to solvent removal by evaporation under reduced pressure in a 40°C water bath to obtain 7.3 g of ethyl 2-((4-((S)-2,2-dimethyltetrahydro-2H-pyran-4-yl)pyridin-2-yl)(hydroxy)methyl)acrylate. The product was transferred to a 250-mL three-necked flask, and then 40 mL of dichloromethane and 2.7 g of pyridine were added. Subsequently, the mixture was cooled to 0°C in an ice-water bath, and 2.7 g of acetyl chloride was added dropwise. Then the reaction was carried out at room temperature for 1 hour. After the completion of the reaction as detected by LCMS, the reaction mixture was quenched by pouring into an ice-cold saturated aqueous sodium bicarbonate solution and stirred at room temperature for half an hour. The mixture was extracted with DCM and washed with saturated brine. The organic phase was collected, dried over anhydrous sodium sulfate, filtered, and subjected to solvent removal by evaporation under reduced pressure in a 40°C water bath to obtain a crude residue. The crude residue was dissolved in 100 mL of toluene and refluxed for 16 hours. After the completion of the reaction as detected by LCMS, the mixture was subjected to solvent removal by evaporation under reduced pressure in a 40°C water bath, and purified by silica gel column chromatography (n-heptane/ethyl acetate = 3:1) to obtain 3.87 g of ethyl (S)-7-(2,2-dimethyltetrahydro-2H-pyran-4-yl)indolizine-2-carboxylate (IX) (yield: 55.3%, purity: 95.5%).

It can be seen that in the present invention, a cyclic anhydride of formula I or a symmetric dicarboxylate of formula II synthesized by the method in the literature undergoes catalytic alcoholysis to obtain an intermediate of formula III with a target stereoconfiguration and a high enantiomeric excess (ee) value. The intermediate reacts with a methyl metal reagent to obtain a lactone of formula IV. The lactone of formula IV reacts with a methyl metal reagent to obtain a cyclized dimethylated compound of formula V-A or formula V-B. The compound of formula V-A undergoes a copper-catalyzed reaction to obtain a corresponding arylhydrazine of formula VI, which is finally subjected to Fisher indole cyclization with a pyruvic acid derivative under acid catalysis to obtain a target product of formula VII. Alternatively, the compound of formula V-B undergoes functional group transformation to obtain an aldehyde of formula VIII, and the latter undergoes a Balis-Hillman reaction with an acrylic acid derivative followed by cyclization to generate a compound of formula IX.

According to the present invention, use of transition metal catalysts and SFC resolution reported in previous methods are avoided, reducing reaction costs and facilitating scale-up production, which represents an economical, environmentally friendly technical route with simple post-treatment and easy for scale-up production.

The above are only preferred examples of the present invention, and do not impose any formal or substantial limitations on the present invention. It should be pointed out that for those of ordinary skill in the art, without departing from the method of the present invention, several improvements and supplements can also be made, and these improvements and supplements shall also be regarded as the scope of protection of the present invention. Any person skilled in the art, without departing from the spirit and scope of the present invention, can utilize the technical content disclosed above to make some equivalent changes such as alterations, modifications and evolutions, all of which are equivalent examples of the present invention. Meanwhile, any equivalent changes such as alterations, modifications and evolutions made to the above examples according to the essential technology of the present invention still belong to the scope of the technical solutions of the present invention.

## Claims

1. A method for synthesizing an aryl-substituted chiral tetrahydropyran ring via desymmetrization, comprising the following steps:
a) alcoholyzing a compound of formula I through organic small-molecule catalysis or enzyme catalysis to obtain a compound of formula III with high chiral purity, wherein a catalyst is selected from 1-((1R,2R)-2-(dimethylamino)cyclohexyl)-3-(4-(trifluoromethyl)phenyl)thiourea, 1-(3,5-bis(trifluoromethyl)phenyl)-3-((1R,2R)-2-(dimethylamino)cyclohexyl)thiourea, 1-(3,5-bis(trifluoromethyl)phenyl)-3-((S)-(6-methoxyquinolin-4-yl)((1S,2S,4S,5R)-5-vinylquinolin-2-yl)methyl)thiourea, N-((S)-(6-methoxyquinolin-4-yl)((1S,2S,4S,5R)-5-vinylquinolin-2-yl)methyl)-3,5-bis(trifluoromethyl)benzenesulfonamide, 1-((1R,2R)-2-(dimethylamino)cyclohexyl)-3-phenylthiourea, or Novozym 435 lipase;
b) reacting the compound of formula III with a methyl metal reagent to obtain a compound of formula IV;
c) reducing a lactone in the compound of formula IV to obtain a compound of formula V;
d) subjecting a compound of formula V-A to a copper-catalyzed hydrazination reaction to obtain a compound of formula VI;
e) subjecting the compound of formula VI to acid-catalyzed Fisher cyclization with a pyruvic acid derivative to obtain a compound of formula VII;
or step d) being replaced with step f):
f) allowing a compound of formula V-B to be treated with a metal reagent and react with N,N-dimethylformamide to obtain a compound of formula VIII; and
g) subjecting the compound of formula VIII to cyclization with an acrylic acid derivative to obtain a compound of formula IX;
wherein the compound of formula I is: 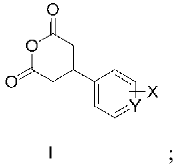
the compound of formula III is: 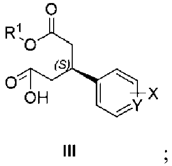
the compound of formula IV is: 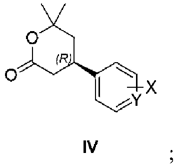
the compound of formula V is: 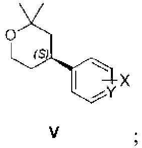
the compound of formula V-A is: 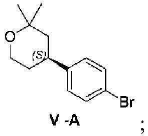
the compound of formula VI is: 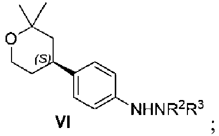
the compound of formula VII is: 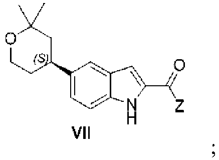
the compound of formula V-B is: 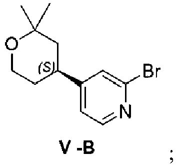
the compound of formula VIII is: 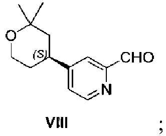 and
the compound of formula IX is: 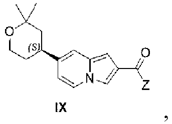
wherein in the formulas, X is Cl, Br, I, -OTf, -OMs, or -OTs; Y is C or N; R¹ is C1-C6 alkyl or benzyl; R² is H, Me, Et, iPr, or Bn; R³ is H or Boc; Z is -OR⁴; R⁴ is C1-C6 alkyl or -NR⁵R⁶; R⁵ is C1-C3 alkyl; and R⁶ is phenyl or substituted phenyl.

2. The method for synthesizing an aryl-substituted chiral tetrahydropyran ring via desymmetrization according to claim 1, wherein the step a) is replaced with step a1): monohydrolyzing a compound of formula II through organic small-molecule catalysis or enzyme catalysis to obtain the compound of formula III with high chiral purity; and
the compound of formula II is: 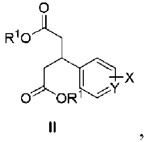
wherein in the formula, X is Cl, Br, I, -OTf, -OMs, or -OTs; Y is C or N; and R¹ is C1-C6 alkyl or benzyl.

3. The method for synthesizing an aryl-substituted chiral tetrahydropyran ring via desymmetrization according to claim 1, wherein in the step a), a substrate for the alcoholysis reaction is one of methanol, ethanol, benzyl alcohol, or other alkyl alcohols, and a quantity of the substrate as used in the alcoholysis reaction is 1.0-10.0 equivalents.

4. The method for synthesizing an aryl-substituted chiral tetrahydropyran ring via desymmetrization according to claim 1 or 2, wherein in the step b), the methyl metal reagent is one of methyllithium, methylmagnesium bromide, methylmagnesium chloride, or methylmagnesium iodide; and a quantity of the methyl metal reagent as used is 3.0-5.0 equivalents.

5. The method for synthesizing an aryl-substituted chiral tetrahydropyran ring via desymmetrization according to claim 1 or 2, wherein in the step c), a reducing agent used for reducing the lactone is DIBAL-H and Et₃SiH.

6. The method for synthesizing an aryl-substituted chiral tetrahydropyran ring via desymmetrization according to claim 1 or 2, wherein in the step d), a copper catalyst is CuI or CuBr, and a quantity of the copper catalyst as used is 0.01-1.0 equivalents.

7. The method for synthesizing an aryl-substituted chiral tetrahydropyran ring via desymmetrization according to claim 1 or 2, wherein in the step d), a ligand used in the reaction is N-(2,6-dimethylphenyl)-6-hydroxypyridinecarboxamide or N1,N2-bis(2,5-dimethyl-1H-pyrrol-1-yl)oxalamide; and a substrate for the hydrazination reaction is hydrazine hydrate, 1-tert-butoxycarbonyl-1-methylhydrazine, 1-tert-butoxycarbonyl-1-ethylhydrazine, or 1-tert-butoxycarbonyl-1-benzylhydrazine.

8. The method for synthesizing an aryl-substituted chiral tetrahydropyran ring via desymmetrization according to claim 1 or 2, wherein in the step e), an acid used for the acid catalysis is HCl, H₂SO₄, PPA, TsOH, or TfOH; and the pyruvic acid derivative is ethyl pyruvate, methyl pyruvate, or N-methyl-2-oxo-N-phenylpropanamide.

9. The method for synthesizing an aryl-substituted chiral tetrahydropyran ring via desymmetrization according to claim 1 or 2, wherein in the step f), the metal reagent is n-butyllithium, sec-butyllithium, isopropylmagnesium chloride, or isopropylmagnesium bromide.

10. The method for synthesizing an aryl-substituted chiral tetrahydropyran ring via desymmetrization according to claim 1 or 2, wherein in the step g), the acrylic acid derivative is ethyl acrylate, methyl acrylate, benzyl acrylate, or N-methyl-N-phenylacrylamide.
